# EUROPÄISCHE PATENTSCHRIFT

(11) **EP 3 229 161 B1**
(45) Veröffentlichungstag und Bekanntmachung des Hinweises auf die Patenterteilung: **24.06.2020**
(21) Anmeldenummer: 17182993.0
(22) Anmeldetag: 25.07.2017
(51) Int. Cl.: G16H 40/63, G16H 30/20

(54) **SYSTEM MIT EINEM MOBILEN STEUERUNGSGERÄT UND VERFAHREN ZUM AUSGEBEN EINES STEUERSIGNALS AN EINE KOMPONENTE EINER MEDIZINISCHEN BILDGEBUNGSVORRICHTUNG**
SYSTEM WITH A MOBILE CONTROL APPARATUS AND METHOD FOR ISSUING A CONTROL SIGNAL TO A COMPONENT OF A MEDICAL IMAGING DEVICE
SYSTÈME COMPRENANT UN APPAREIL DE COMMANDE MOBILE ET PROCÉDÉ D'ÉMISSION D'UN SIGNAL DE COMMANDE SUR UN COMPOSANT D'UN DISPOSITIF D'IMAGERIE MÉDICAL

(30) Priorität: 21.09.2016 DE 102016218138
(43) Veröffentlichungstag der Anmeldung: 11.10.2017
(73) Patentinhaber: Siemens Healthcare GmbH, 91052 Erlangen (DE)
(72) Erfinder: Lerch, Daniel, 91365 Weilersbach (DE); Boettger, Thomas, 91054 Erlangen (DE); Hannemann, Thilo, 91052 Erlangen (DE); Krämer, Gerhard, 91338 Igensdorf (DE); Thierfelder, Carsten, 91361 Pinzberg (DE); Vega, Fernando, 91056 Erlangen (DE); Zehner, Selma, 91301 Forchheim (DE)

(56) Entgegenhaltungen:
- EP-A1- 2 502 558
- WO-A1-2014/070860
- DE-A1-102011 083 957

## Beschreibung

Die Erfindung betrifft ein System, aufweisend eine Komponente einer medizinischen Bildgebungsvorrichtung und ein mobiles Steuerungsgerät. Die Erfindung betrifft ferner ein Verfahren zum Ausgeben eines Steuersignals an eine Komponente einer medizinischen Bildgebungsvorrichtung. Die Erfindung betrifft ferner eine Verwendung eines Systems zum Ausgeben eines Steuersignals an eine Komponente einer medizinischen Bildgebungsvorrichtung.

Medizinische Bildgebungsvorrichtungen, insbesondere schnittbildgebende Medizingeräte wie beispielsweise CT-Geräte und MR-Geräte, erfordern typischerweise eine möglichst genaue Positionierung der Transferplatte, auf der ein Patient gelagert ist, relativ zu der Gantry. In vielen Fällen wird diese Positionierung unter Verwendung von Knöpfen oder anderen Kontrollelementen, welche unmittelbar an der Gantry fest angeordnet sind, vorgenommen. Je nach Gerät befinden sich diese Knöpfe oder andere Kontrollelemente ausschließlich an der Vorderseite oder sowohl an der Vorderseite als auch an der Hinterseite des Geräts. Oft sind die Kontrollelemente redundant an mehreren Stellen auf der Gantry verteilt angeordnet, was einen negativen Einfluss in Bezug auf Kosten und Komplexität zur Folge hat. Je nach den Bedürfnissen des Patienten und kulturellen Gegebenheiten kann die Beschränkung, die Lagerung und/oder die Entlagerung des Patienten nur stehend direkt neben dem Patienten durchführen zu können, eine starke Einschränkung darstellen. So wäre es beispielsweise aus Gründen des Zeitgewinns oder auch der Patientensicherheit, insbesondere im Falle eines Notfalls, wünschenswert, die Transferplatte bereits dann bewegen zu können, wenn der Patient und/oder der Nutzer den Untersuchungsraum betritt. Im Falle von kulturellen oder gesundheitlichen Einschränkungen kann es ebenfalls sinnvoll sein die Patientenlagerungsvorrichtung von einer Position aus zu steuern, die weiter von der medizinischen Bildgebungsvorrichtung entfernt ist.

WO 2014/070860 A1 offenbart ein medizinisches Bildgebungssystem, umfassend eine Bildgebungsquelle, einen Bildgebungsdetektor und eine tragbare Detektorsteuervorrichtung.

DE 10 2011 083957 A offenbart ein elektronisches, kontextsensitives Steuerungssystem für ein medizintechnisches Gerät mit zumindest einem externen Ein- und Ausgabegerät mit einer Touchscreen-Benutzeroberfläche.

EP 2 502 558 A1 offenbart einen medizinischen Arbeitsplatz, aufweisend ein medizinisches Instrument für eine Behandlung eines Lebewesens, eine bildgebende Vorrichtung, um während der Behandlung Bilddatensätze vom Inneren des Lebewesens zu erstellen, und einen Roboter.

Die Erfindung hat die Aufgabe, eine alternative Möglichkeit zur Steuerung einer Komponente einer medizinischen Bildgebungsvorrichtung bereitzustellen.

Jeder der Gegenstände der unabhängigen Ansprüche löst jeweils diese Aufgabe. In den abhängigen Ansprüchen sind weitere vorteilhafte Aspekte der Erfindung berücksichtigt.

Die Erfindung betrifft ein System, aufweisend
- eine Komponente einer medizinischen Bildgebungsvorrichtung,
- ein mobiles Steuerungsgerät mit einem Bedienelement, welches als ein elektromechanisches Schaltelement ausgebildet ist,
- eine Steuerungseinheit, welche zum Ausgeben eines Steuersignals an die Komponente ausgebildet ist,
- eine Posen-Ermittlungseinheit, welche zum Ermitteln einer Pose des mobilen Steuerungsgeräts ausgebildet ist,
- eine Konfigurationseinheit, welche zum Konfigurieren des mobilen Steuerungsgeräts und/oder der Steuerungseinheit basierend auf der Pose des mobilen Steuerungsgeräts ausgebildet ist,- wobei in einem Betriebszustand des Systems das mobile Steuerungsgerät und die Steuerungseinheit derart gekoppelt sind, dass ein Betätigen des Bedienelements das Ausgeben des Steuersignals an die Komponente mittels der Steuerungseinheit bewirkt, - wobei die Komponente der medizinischen Bildgebungsvorrichtung einen Lagerungstisch und eine Transferplatte aufweist, welche relativ zu dem Lagerungstisch bewegbar an dem Lagerungstisch angeordnet ist,
- wobei das mobile Steuerungsgerät ein erstes Transfer-Bedienelement aufweist,
- wobei das mobile Steuerungsgerät und/oder die Steuerungseinheit mittels der Konfigurationseinheit derart basierend auf der Pose des mobilen Steuerungsgeräts konfigurierbar ist, dass für zumindest eine erste Pose des mobilen Steuerungsgeräts ein Betätigen des ersten Transfer-Bedienelements ein Bewegen der Transferplatte in eine erste horizontale Richtung bewirkt und dass für zumindest eine zweite Pose des mobilen Steuerungsgeräts ein Betätigen des ersten Transfer-Bedienelements ein Bewegen der Transferplatte in eine zweite horizontale Richtung bewirkt.

Das mobile Steuerungsgerät kann insbesondere derart ausgebildet sein, dass das mobile Steuerungsgerät von einem Nutzer in einer Hand und/oder am Körper getragen werden kann und/oder dass das mobile Steuerungsgerät von einem Nutzer bedient werden kann, während das mobile Steuerungsgerät von dem Nutzer in einer Hand und/oder am Körper getragen wird.

Das mobile Steuerungsgerät kann insbesondere eine erste Audio-Schnittstelle aufweisen, welche dazu ausgebildet ist, akustische Signale von dem Nutzer zu empfangen und/oder an den Nutzer auszugeben.

Das mobile Steuerungsgerät kann insbesondere mehrere berührungsempfindliche Bedienelemente aufweisen, welche zusammen eine berührungsempfindliche Fläche bilden, welche zum Anzeigen zumindest eines Software-Bedienelements und/oder zum Erfassen zumindest einer Berührungsgeste zum Betätigen zumindest eines Software-Bedienelements ausgebildet ist.

Das mobile Steuerungsgerät kann insbesondere eine Anzeigeeinheit aufweisen, welche dazu ausgebildet ist, dem Nutzer Daten, welche die medizinische Bildgebungsvorrichtung und/oder die Komponente der medizinischen Bildgebungsvorrichtung betreffen, anzuzeigen.

Das System kann insbesondere ferner die folgenden Komponenten aufweisen:
- zumindest eine Kontext-Ermittlungseinheit, welche zum Ermitteln eines Kontexts ausgebildet ist, welcher die medizinische Bildgebungsvorrichtung und/oder eine medizinische Untersuchung, welche mittels der medizinischen Bildgebungsvorrichtung durchführbar und/oder zumindest teilweise durchgeführt ist, betrifft.

Die Posen-Ermittlungseinheit kann insbesondere dazu ausgebildet sein, zu ermitteln, ob die Pose des mobilen Steuerungsgeräts eine Posenbereichsgrenze überschreitet.

Das System kann insbesondere eine Alarmsignal-Ausgabeeinheit aufweisen, welche zum Ausgeben eines Alarmsignals derart ausgebildet ist, dass das Alarmsignal ausgegeben wird, wenn die Pose des mobilen Steuerungsgeräts eine Posenbereichsgrenze überschreitet.

Das System kann insbesondere ferner eine Konfigurationseinheit aufweisen, welche zum Konfigurieren des mobilen Steuerungsgeräts und/oder der Steuerungseinheit basierend auf dem Kontext und/oder basierend auf der Pose des mobilen Steuerungsgeräts ausgebildet ist.

Die Posen-Ermittlungseinheit kann insbesondere dazu ausgebildet sein, zu ermitteln, ob sich die Pose des mobilen Steuerungsgeräts in einem ersten Posenbereich befindet,
- wobei das mobile Steuerungsgerät und/oder die Steuerungseinheit mittels der Konfigurationseinheit derart basierend auf der Pose des mobilen Steuerungsgeräts konfigurierbar ist, dass ein Betätigen des Bedienelements das Ausgeben des Steuersignals an die Komponente mittels der Steuerungseinheit in Abhängigkeit davon bewirkt, ob sich die Pose in dem ersten Posenbereich befindet.

Die Posen-Ermittlungseinheit kann insbesondere dazu ausgebildet sein, zu ermitteln, ob sich die Pose des mobilen Steuerungsgeräts in einem zweiten Posenbereich befindet,
- wobei das mobile Steuerungsgerät und/oder die Steuerungseinheit mittels der Konfigurationseinheit derart basierend auf der Pose des mobilen Steuerungsgeräts konfigurierbar ist, dass das mobile Steuerungsgerät und die Steuerungseinheit in Abhängigkeit davon gekoppelt sind, ob sich die Pose in dem zweiten Posenbereich befindet.

Das System kann insbesondere einen Tabletcomputer mit einer Software-Applikation, welche zum Steuern der medizinischen Bildgebungsvorrichtung und/oder der Komponente der medizinischen Bildgebungsvorrichtung ausgebildet ist, aufweisen,
- wobei das mobile Steuerungsgerät den Tabletcomputer aufweist und/oder wobei das mobile Steuerungsgerät mit dem Tabletcomputer mittels einer mechanischen Verbindung und/oder mittels einer Datenübertragungsverbindung verbindbar und/oder verbunden ist.

Das System kann insbesondere ein Smartphone mit einer Software-Applikation, welche zum Steuern der medizinischen Bildgebungsvorrichtung und/oder der Komponente der medizinischen Bildgebungsvorrichtung ausgebildet ist, aufweisen,
- wobei das mobile Steuerungsgerät das Smartphone aufweist und/oder wobei das mobile Steuerungsgerät mit dem Smartphone mittels einer mechanischen Verbindung und/oder mittels einer Datenübertragungsverbindung verbindbar und/oder verbunden ist.

Das System kann insbesondere die medizinische Bildgebungsvorrichtung aufweisen, wobei die medizinische Bildgebungsvorrichtung eine Andockeinheit aufweist, welche zum lösbaren Andocken des mobilen Steuerungsgeräts ausgebildet ist.

Insbesondere kann die mechanische Verbindung und/oder die Andockeinheit eine magnetische Halterung aufweisen.

Die Erfindung betrifft ferner ein Verfahren zum Ausgeben eines Steuersignals an eine Komponente einer medizinischen Bildgebungsvorrichtung,
wobei das Verfahren die folgenden Schritte umfasst:
- Koppeln eines mobilen Steuerungsgeräts, welches ein Bedienelement aufweist, welches als ein elektromechanisches Schaltelement ausgebildet ist, und
   einer Steuerungseinheit, welche zum Ausgeben eines Steuersignals an die Komponente ausgebildet ist, derart, dass ein Betätigen des Bedienelements das Ausgeben des Steuersignals an die Komponente mittels der Steuerungseinheit bewirkt,
- Betätigen des Bedienelements,
- Ausgeben des Steuersignals,
- Ermitteln einer Pose des mobilen Steuerungsgeräts,
- Konfigurieren des mobilen Steuerungsgeräts und/oder der Steuerungseinheit basierend auf der Pose des mobilen Steuerungsgeräts,
- wobei die Komponente der medizinischen Bildgebungsvorrichtung einen Lagerungstisch und eine Transferplatte aufweist, welche relativ zu dem Lagerungstisch bewegbar an dem Lagerungstisch angeordnet ist,
- wobei das mobile Steuerungsgerät ein erstes Transfer-Bedienelement aufweist,
- wobei das mobile Steuerungsgerät und/oder die Steuerungseinheit mittels der Konfigurationseinheit derart basierend auf der Pose des mobilen Steuerungsgeräts konfigurierbar ist, dass für zumindest eine erste Pose des mobilen Steuerungsgeräts ein Betätigen des ersten Transfer-Bedienelements ein Bewegen der Transferplatte in eine erste horizontale Richtung bewirkt und dass für zumindest eine zweite Pose des mobilen Steuerungsgeräts ein Betätigen des ersten Transfer-Bedienelements ein Bewegen der Transferplatte in eine zweite horizontale Richtung bewirkt.

Das Verfahren kann insbesondere ferner die folgenden Schritte umfassen:
- Ermitteln eines Kontexts, welcher die medizinische Bildgebungsvorrichtung und/oder eine medizinische Untersuchung, welche mittels der medizinischen Bildgebungsvorrichtung durchführbar und/oder zumindest teilweise durchgeführt ist, betrifft,
- Konfigurieren des mobilen Steuerungsgeräts und/oder der Steuerungseinheit basierend auf dem Kontext.

Die Erfindung betrifft ferner die Verwendung eines Systems nach einem der Aspekte, die in dieser Beschreibung und/oder in den Ansprüchen offenbart sind, zum Ausgeben eines Steuersignals an eine Komponente einer medizinischen Bildgebungsvorrichtung, wobei das Ausgeben des Steuersignals an die Komponente mittels der Steuerungseinheit bewirkt wird, indem das Bedienelement betätigt wird.

Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus (insbesondere PET-CT-Gerät, PET-MR-Gerät) besteht. Die medizinische Bildgebungsvorrichtung kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen. Ohne Einschränkung des allgemeinen Erfindungsgedankens wird bei einigen der Ausführungsformen ein Computertomographiegerät beispielhaft für eine medizinische Bildgebungsvorrichtung genannt.

Gemäß einer Ausführungsform der Erfindung weist die medizinische Bildgebungsvorrichtung eine Akquisitionseinheit, welche zur Akquisition der Akquisitionsdaten ausgebildet ist, auf. Insbesondere kann die Akquisitionseinheit eine Strahlungsquelle und einen Strahlungsdetektor aufweisen. Eine Ausführungsform der Erfindung sieht vor, dass die Strahlungsquelle zur Emission und/oder zur Anregung einer Strahlung, insbesondere einer elektromagnetischen Strahlung, ausgebildet ist und/oder dass der Strahlungsdetektor zur Detektion der Strahlung, insbesondere der elektromagnetischen Strahlung, ausgebildet ist. Die Strahlung kann beispielsweise von der Strahlungsquelle zu einem abzubildenden Bereich gelangen und/oder nach einer Wechselwirkung mit dem abzubildenden Bereich zu dem Strahlungsdetektor gelangen. Bei der Wechselwirkung mit dem abzubildenden Bereich wird die Strahlung modifiziert und damit zum Träger von Informationen, die den abzubildenden Bereich betreffen. Bei der Wechselwirkung der Strahlung mit dem Detektor werden diese Informationen in Form von Akquisitionsdaten erfasst.

Insbesondere bei einem Computertomographiegerät und bei einem C-Bogen-Röntgengerät können die Akquisitionsdaten Projektionsdaten, die Akquisitionseinheit eine Projektionsdaten-Akquisitionseinheit, die Strahlungsquelle eine Röntgenquelle, der Strahlungsdetektor ein Röntgendetektor sein. Der Röntgendetektor kann insbesondere ein quantenzählender und/oder energieauflösender Röntgendetektor sein. Insbesondere bei einem Magnetresonanztomographiegerät können die Akquisitionsdaten ein Magnetresonanzdatensatz, die Akquisitionseinheit eine Magnetresonanzdaten-Akquisitionseinheit, die Strahlungsquelle eine erste Hochfrequenzantenneneinheit, der Strahlungsdetektor die erste Hochfrequenzantenneneinheit und/oder eine zweite Hochfrequenzantenneneinheit sein.

Die Komponente der medizinischen Bildgebungsvorrichtung kann beispielsweise eine Komponente einer Patientenlagerungsvorrichtung der medizinischen Bildgebungsvorrichtung und/oder eine Komponente einer Gantry der medizinischen Bildgebungsvorrichtung sein. Eine Komponente der Patientenlagerungsvorrichtung kann beispielsweise ein Antrieb sein, der zum Antreiben einer Bewegung der Transferplatte relativ zu dem Lagerungstisch ausgebildet ist. Eine Komponente der Gantry kann beispielsweise ein Laservisier zur Positionierung des Patienten sein und/oder eine Strahlungsquelle sein. Die Komponente kann insbesondere dazu ausgebildet sein, das Steuersignal zu empfangen. Bei einer Patientenlagerungsvorrichtung kann ein Steuersignal beispielsweise eine Bewegung der Transferplatte bewirken. Bei einer Strahlungsquelle kann ein Steuersignal beispielsweise ein Auslösen einer Strahlung bewirken.

Das mobile Steuerungsgerät kann insbesondere ein Gehäuse aufweisen. Das Gehäuse kann insbesondere derart ausgebildet sein, dass durch das Gehäuse ein Innenraum des mobilen Steuerungsgeräts definiert, insbesondere in alle Richtungen begrenzt, ist.

Das System kann beispielsweise eine Trageeinheit aufweisen, welche zum Tragen des mobilen Steuerungsgeräts am Körper eines Nutzers ausgebildet ist. Die Trageeinheit kann beispielsweise ein Element aufweisen, welches aus der Gruppe gewählt ist, welche aus einem Armband, einem Gürtel, einem Lanyard und Kombinationen davon besteht. Die Trageinheit kann beispielsweise ferner ein Verbindungselement aufweisen, welches zum insbesondere lösbaren Verbinden mit dem Gehäuse und/oder mit dem Andockmodul des mobilen Steuerungsgeräts ausgebildet ist. Das mobile Steuerungsgerät kann insbesondere dann, wenn es die Trageeinheit aufweist und/oder mit der Trageeinheit verbunden ist, als ein Wearable ausgebildet sein.

Das System kann insbesondere einen weiteren Betriebszustand aufweisen, in dem das mobile Steuerungsgerät und die Steuerungseinheit nicht gekoppelt sind oder nicht derart gekoppelt sind, dass ein Betätigen des Bedienelements das Ausgeben des Steuersignals an die Komponente mittels der Steuerungseinheit bewirkt. Insbesondere können in dem weiteren Betriebszustand des Systems das mobile Steuerungsgerät und die Steuerungseinheit derart koppelbar sein, dass ein Betätigen des Bedienelements das Ausgeben des Steuersignals an die Komponente mittels der Steuerungseinheit bewirkt. Das mobile Steuerungsgerät und die Steuerungseinheit können insbesondere zumindest teilweise mittels Hardware und/oder zumindest teilweise mittels Firmware und/oder zumindest teilweise mittels Software gekoppelt sein. Die Steuerungseinheit kann insbesondere in eine Steuerungsvorrichtung der medizinischen Bildgebungsvorrichtung integriert sein und/oder an der Komponente der medizinischen Bildgebungsvorrichtung angeordnet sein.

Ein Kontext, welcher die medizinische Bildgebungsvorrichtung und/oder eine medizinische Untersuchung, welche mittels der medizinischen Bildgebungsvorrichtung durchführbar und/oder zumindest teilweise durchgeführt ist, betrifft, kann insbesondere eine Information aufweisen, welche einen Zustand der medizinischen Bildgebungsvorrichtung und/oder der Komponente der medizinischen Bildgebungsvorrichtung und/oder einen Stand der Untersuchung, beispielsweise bereits ausgeführte Schritte der Untersuchung und/oder noch auszuführende Schritte der Untersuchung, betrifft.

Ein Kontext, der eine Patientenlagerungsvorrichtung betrifft, kann beispielsweise eine Information aufweisen, ob die Transferplatte in die tunnelförmige Öffnung eingeführt ist oder nicht. Ein Kontext, der die Untersuchung betrifft, kann beispielsweise eine Information aufweisen, dass bei dem nächsten Schritt des vorgegebenen Workflows die Transferplatte in die tunnelförmige Öffnung einzuführen ist oder dass bei dem nächsten Schritt des vorgegebenen Workflows die Strahlung für die Akquisition von Akquisitionsdaten auszulösen ist.

Eine Pose des mobilen Steuerungsgeräts kann insbesondere eine Information aufweisen, welche eine Position des mobilen Steuerungsgeräts und/oder eine Orientierung des mobilen Steuerungsgeräts betrifft. Die Pose des mobilen Steuerungsgeräts kann beispielsweise relativ zu einem Untersuchungsraum, in dem die medizinische Bildgebungsvorrichtung installiert ist, und/oder relativ zu einer Komponente der medizinischen Bildgebungsvorrichtung definiert sein.

Unter einer Position des mobilen Steuerungsgeräts kann beispielsweise ein Abstand des mobilen Steuerungsgeräts zu einem Referenzort, beispielsweise an der Gantry, verstanden werden. Insbesondere kann das mobile Steuerungsgerät und/oder die Steuerungseinheit mittels der Konfigurationseinheit derart basierend auf der Pose des mobilen Steuerungsgeräts konfigurierbar sein, dass sicherheitsrelevante Funktionen nicht mehr ausgelöst werden können, wenn der Abstand zum Referenzort einen Schwellwert überschreitet. Das Ermitteln der Pose kann beispielsweise basierend auf einer Triangulation, einer Feldstärkemessung oder einer Laufzeitmessung eines Datenübertragungsmediums insbesondere bei einer IR- und/oder funkbasierten Datenübertragung, erfolgen. Insbesondere in Bezug auf die Posenbereichsgrenze kann das Ermitteln der Pose beispielsweise auf einer Detektion eines elektronischen Schwingkreises und/oder Schaltkreises mittels eines Antennensystems basieren.

Ferner kann das Ermitteln der Pose beispielsweise basierend auf einer Ermittlung der Andockeinheit, an welche das mobile Steuerungsgerät angedockt ist, erfolgen. Diese Möglichkeit kann insbesondere dann sinnvoll sein, wenn die medizinische Bildgebungsvorrichtung und/oder die Umgebung der medizinischen Bildgebungsvorrichtung mehrere Andockeinheiten, die räumlich verteilt angeordnet sind und deren Positionsinformationen dem System bekannt sind, aufweist.

Das lösbare Andocken des mobilen Steuerungsgeräts an die Andockeinheit kann beispielsweise eine mechanische und/oder formschlüssige Verbindung des mobilen Steuerungsgeräts mit der Andockeinheit bilden, wobei die Verbindung insbesondere derart fest ausgebildet ist, dass die Kräfte und/oder Momente, die beim Betätigen der Bedienelemente auf das mobile Steuerungsgerät wirken, von der Andockeinheit aufgenommen werden. Auf diese Weise kann der Nutzer das mobile Steuerungsgerät, welches an die Andockeinheit angedockt ist, einhändig bedienen. Das lösbare Andocken des mobilen Steuerungsgeräts an die Andockeinheit kann beispielsweise eine Energieübertragungsverbindung und/oder eine Datenübertragungsverbindung zwischen dem mobilen Steuerungsgerät und der Andockeinheit bilden. Beispielsweise kann das mobile Steuerungsgerät kabellos an der Andockeinheit mit elektrischer Energie aufgeladen werden.

Die erfindungsgemäße Lösung ermöglicht dem Nutzer einer medizinischen Bildgebungsvorrichtung eine weitgehende Autonomie darüber, wie, wann und von wo aus er seinen Workflow gestaltet. Damit kann der Nutzer den Workflow beispielsweise besser an die Bedürfnisse des Patienten anpassen. Beispielsweise kann der Nutzer seine Position und Orientierung relativ zu der Gantry und/oder relativ zu der Patientenlagerungsvorrichtung während der Untersuchung weitgehend unabhängig wählen und flexibel anpassen. Ferner kann der Nutzer das mobile Steuerungsgerät bedienen, während er geht oder läuft, was insbesondere für Notfall-Situationen einen wertvollen Zeitvorteil ermöglicht.

Bedienelemente, die als elektromechanisches Schaltelement ausgebildet sind, ermöglichen zudem ein höheres Maß an Sicherheit, insbesondere in Bezug auf Bewegungen der Transferplatte und der Auslösung der Strahlung, als beispielsweise Bedienelement, die in eine Software-Applikation eingebettet sind.

Das Bedienelement kann insbesondere dann als elektromechanisches Schaltelement ausgebildet sein, wenn es das elektromechanische Schaltelement aufweist. Das elektromechanische Schaltelement kann insbesondere ein Schalter, beispielsweise ein Taster, sein.

Das Betätigen des Bedienelements kann insbesondere ein Betätigen des elektromechanischen Schaltelements umfassen. Das Betätigen des elektromechanischen Schaltelements kann insbesondere einen mechanischen Schaltvorgang umfassen, bei dem beispielsweise eine elektrisch leitende Verbindung hergestellt und/oder getrennt wird und/oder bei dem beispielsweise zumindest ein mechanischer und/oder geometrischer Parameter eines elektronischen Schaltkreises verändert wird.

Insbesondere können in dem Betriebszustand des Systems das mobile Steuerungsgerät und die Steuerungseinheit derart gekoppelt sein, dass ein Betätigen des elektromechanischen Schaltelements das Ausgeben des Steuersignals an die Komponente mittels der Steuerungseinheit bewirkt.

Das Bedienelement kann insbesondere das elektromechanische Schaltelement und/oder eine berührungsempfindliche Oberfläche aufweisen. Die berührungsempfindliche Oberfläche des Bedienelements kann insbesondere einen Teil der berührungsempfindlichen Fläche bilden.

Insbesondere kann dem Bedienelement basierend auf einer Berührungsgeste, welche mittels der berührungsempfindlichen Oberfläche des Bedienelements und/oder mittels der berührungsempfindlichen Fläche erfasst wird, die Komponente und/oder eine Funktion zugeordnet werden. Insbesondere kann ein Betätigen des Bedienelements und/oder ein Betätigen des elektromechanischen Schaltelements das Ausgeben des Steuersignals an die Komponente mittels der Steuerungseinheit bewirken, wobei das Steuersignal die Funktion, die dem Bedienelement zugeordnet ist, betrifft.

Insbesondere kann eine Komponente eines Systems nach einem der Aspekte, die in dieser Beschreibung und/oder in den Ansprüchen offenbart sind, welche dazu ausgebildet ist, einen gegebenen Schritt eines Verfahrens nach einem der Aspekte, die in dieser Beschreibung und/oder in den Ansprüchen offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Insbesondere kann das System einen Speicherbereich, beispielsweise in Form eines computerlesbaren Mediums, aufweisen, in welchem computerlesbare Anweisungen, beispielsweise in Form eines Computerprogramms, gespeichert sind.

Bei der Hardware kann es sich beispielsweise um ein Speichersystem, ein FPGA-System (Field-programmable gate array), ein ASIC-System (Application-specific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Die Verwendung von Ordnungszahlwörtern (erste, zweite, dritte etc.) in der Bezeichnung von Merkmalen dient im Kontext der vorliegenden Anmeldung vor allem der besseren Unterscheidbarkeit der unter Verwendung von Ordnungszahlwörtern bezeichneten Merkmale. Das Nichtvorhandensein eines Merkmals, welches durch eine Kombination eines gegebenen Ordnungszahlworts und eines Begriffs bezeichnet wird, schließt nicht aus, dass ein Merkmal vorhanden sein kann, welches durch eine Kombination eines dem gegebenen Ordnungszahlwort nachfolgenden Ordnungszahlworts und des Begriffs bezeichnet wird.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden werden ausgewählte Ausführungsformen unter Hinweis auf die beigefügten Figuren erläutert. Die Darstellung in den Figuren ist schematisch, stark vereinfacht und nicht zwingend maßstabsgetreu.

Es zeigen:
Fig. 1 ein mobiles Steuerungsgerät eines Systems gemäß einer ersten Ausführungsform der Erfindung,
Fig. 2 ein mobiles Steuerungsgerät eines Systems gemäß einer zweiten Ausführungsform der Erfindung in einer ersten Ansicht,
Fig. 3 ein mobiles Steuerungsgerät eines Systems gemäß der zweiten Ausführungsform der Erfindung in einer zweiten Ansicht,
Fig. 4 ein mobiles Steuerungsgerät eines Systems gemäß einer dritten Ausführungsform der Erfindung,
Fig. 5 ein System gemäß einer vierten Ausführungsform der Erfindung,
Fig. 6 ein System gemäß einer fünften Ausführungsform der Erfindung,
Fig. 7 ein System gemäß einer sechsten Ausführungsform der Erfindung,
Fig. 8 ein System gemäß einer siebenten Ausführungsform der Erfindung,
Fig. 9 ein Ablaufdiagramm für ein Verfahren zum Ausgeben eines Steuersignals an eine Komponente einer medizinischen Bildgebungsvorrichtung gemäß einer achten Ausführungsform der Erfindung,
Fig. 10 ein Ablaufdiagramm für ein Verfahren zum Ausgeben eines Steuersignals an eine Komponente einer medizinischen Bildgebungsvorrichtung gemäß einer neunten Ausführungsform der Erfindung.

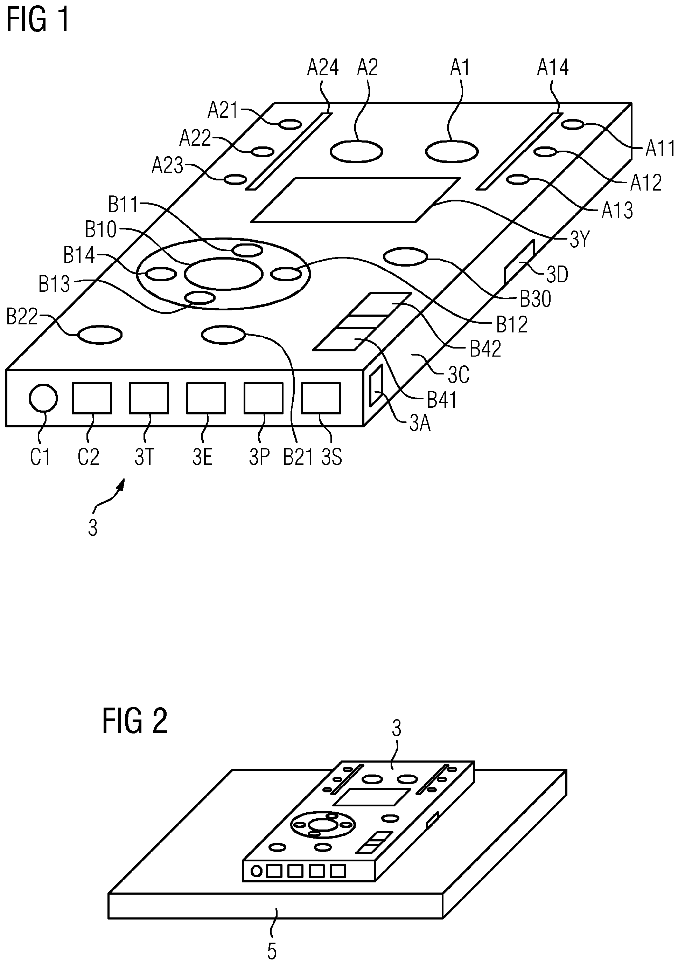

Fig. 1 zeigt ein mobiles Steuerungsgerät 3 eines Systems 1 gemäß einer ersten Ausführungsform der Erfindung.

Das mobile Steuerungsgerät 3 weist ein Gehäuse 3C auf. Das Gehäuse 3C ist derart ausgebildet, dass durch das Gehäuse 3C ein Innenraum des mobilen Steuerungsgeräts 3 definiert, insbesondere in alle Richtungen begrenzt, ist. Das Gehäuse 3C kann beispielsweise flüssigkeitsdicht und/oder flüssigkeitsabweisend ausgebildet sein. Das Gehäuse 3C kann beispielsweise mit antibakteriellen Materialien ausgebildet sein und/oder derart geformt sein, dass Ritzen, die nur mit großem Aufwand desinfizierbar sind, vermieden sind.

Jedes der Bedienelemente B10, B11, B12, B13, B14, B21, B22, B30, B41, B42 ist jeweils als ein elektromechanisches Schaltelement an dem Gehäuse 3C ausgebildet und/oder von einem Nutzer U1 manuell betätigbar. Ein elektromechanisches Schaltelement kann insbesondere einen Taster und/oder einen Schalter aufweisen. Das Bedienen des mobilen Steuerungsgeräts 3 umfasst insbesondere das Betätigen eines oder mehrerer der Bedienelemente.

Das mobile Steuerungsgerät 3 weist einen haptischen Button B10 auf, der kontextsensitiv jeweils den nächsten Schritt eines vorgegebenen Workflows freigibt, aktiviert oder abschließt. Der vorgegebene Workflow kann sowohl Hardware-Aktionen als auch Software-Aktionen umfassen. Beispiele für Hardware-Aktionen sind das Laden und/oder das Entladen der Patientenlagerungsvorrichtung 10 sowie das Starten eines CT-Scans. Beispiele für Software-Aktionen sind das Verschicken der Bilder einer Untersuchung in einem PACS oder das Ausführen des jeweils nächsten Schritts in einer Folge konsekutiver Arbeitsschritte in einer Software-Applikation, welche beispielsweise auf dem Tabletcomputer 5 und/oder auf einem Smartphone und/oder auf der Steuerungsvorrichtung 30 installiert ist.

Ein Betätigen des Bedienelements kann insbesondere bewirken, dass ein Steuersignal an eine Komponente entsprechend der Funktion, die dem Bedienelement zugeordnet ist, ausgegeben wird. Den Bedienelementen und/oder Anzeigeelementen des mobilen Steuerungsgeräts 3 sind insbesondere die folgenden Funktionen zugeordnet:
B11: Heben der Transferplatte 12, B13: Senken der Transferplatte 12, B21: Laden und/oder Entladen der Patientenlagerungsvorrichtung 10, B22: Lichtschalter, B30: Not-AusSchalter, B41: Start der Strahlungsexposition, B42: Stopp der Strahlungsexposition,
A11: Empfindlichkeit des Mikrophons A1 erhöhen,
A12: Empfindlichkeit des Mikrophons A1 reduzieren, A13: Markierung, die dem Nutzer U1 anzeigt, dass in diesem Bereich Bedienelemente und Anzeigeelemente angeordnet sind, die das Mikrophon A1 betreffen, A14: Anzeigen der Empfindlichkeit des Mikrophons A1,
A21: Lautstärke des Lautsprechers A2 erhöhen, A22: Lautstärke des Lautsprechers A2 reduzieren, A23: Markierung, die dem Nutzer U1 anzeigt, dass in diesem Bereich Bedienelemente und Anzeigeelemente angeordnet sind, die den Lautsprecher A2 betreffen, A24: Anzeigen der Lautstärke des Lautsprechers A2.

Das mobile Steuerungsgerät 3 weist ein erstes Transfer-Bedienelement B12 und ein zweites Transfer-Bedienelement B14 auf. Ein Betätigen des zweiten Transfer-Bedienelements B14 bewirkt ein Bewegen der Transferplatte 12 in eine horizontale Richtung, welche entgegengesetzt zu der horizontalen Richtung ist, in welche die Transferplatte 12 bewegt wird, wenn das erste Transfer-Bedienelement B12 betätigt wird. Insbesondere kann die Zuordnung der horizontalen Richtungen zu den Transfer-Bedienelementen B12 und B14 basierend auf der Pose des mobilen Steuerungsgeräts 3 relativ zu der Transferplatte 12 und/oder relativ zu der medizinischen Bildgebungsvorrichtung 2 erfolgen.

Beispielsweise kann das mobile Steuerungsgerät 3 und/oder die Steuerungseinheit 35 mittels der Konfigurationseinheit 7N derart basierend auf der Pose des mobilen Steuerungsgeräts 3 konfigurierbar sein, dass für eine erste Pose des mobilen Steuerungsgeräts 3, ein Betätigen des ersten Transfer-Bedienelements B12 ein Einführen der Transferplatte 12 in die tunnelförmige Öffnung 9 bewirkt und/oder ein Betätigen des zweiten Transfer-Bedienelements B14 ein Bewegen der Transferplatte 12 weg von der tunnelförmige Öffnung 9 bewirkt, und dass für eine zweite Pose des mobilen Steuerungsgeräts 3, ein Betätigen des ersten Transfer-Bedienelements B12 ein Bewegen der Transferplatte 12 weg von der tunnelförmige Öffnung 9 bewirkt und/oder ein Betätigen des zweiten Transfer-Bedienelements B14 ein Einführen der Transferplatte 12 in die tunnelförmige Öffnung 9 bewirkt.

Die erste horizontale Richtung kann beispielsweise von dem Lagerungstisch 11 auf die tunnelförmige Öffnung 9 zeigen. Das Bewegen der Transferplatte 12 in die erste horizontale Richtung kann insbesondere ein Einführen der Transferplatte 12 in die tunnelförmige Öffnung 9 umfassen. Die zweite horizontale Richtung kann beispielsweise von der tunnelförmigen Öffnung 9 auf den Lagerungstisch 11 zeigen. Das Bewegen der Transferplatte 12 in die zweite horizontale Richtung kann insbesondere ein Bewegen der Transferplatte 12 weg von der tunnelförmigen Öffnung 9 umfassen.

Insbesondere kann es sich bei der ersten Pose um eine Pose handeln, bei der sich das mobile Steuerungsgerät 3 in Bezug auf die Gantry 20 auf derselben Seite befindet wie die Patientenlagerungsvorrichtung 10 und bei der das mobile Steuerungsgerät 3 derart orientiert ist, dass sich das erste Transfer-Bedienelement B12 näher an der Gantry 20 befindet als das zweite Transfer-Bedienelement B14.
Insbesondere kann es sich bei der zweiten Pose um eine Pose handeln, bei der sich das mobile Steuerungsgerät 3 in Bezug auf die Gantry 20 auf derselben Seite befindet wie die Patientenlagerungsvorrichtung 10 und bei der das mobile Steuerungsgerät 3 derart orientiert ist, dass sich das zweite Transfer-Bedienelement B14 näher an der Gantry 20 befindet als das erste Transfer-Bedienelement B12.

Die erste Pose des mobilen Steuerungsgeräts 3 kann beispielsweise dann vorliegen, wenn sich das erste Transfer-Bedienelement B12 aus der Sicht des Nutzers U1 rechts von dem zweiten Transfer-Bedienelement B14 befindet, der Nutzer U1 der Patientenlagerungsvorrichtung 10 und dem mobilen Steuerungsgerät 3 zugewandt ist und sich die Gantry 20 zur rechten Hand des Nutzers U1 befindet. Die zweite Pose des mobilen Steuerungsgeräts 3 kann beispielsweise dann vorliegen, wenn sich das erste Transfer-Bedienelement B12 aus der Sicht des Nutzers U1 rechts von dem zweiten Transfer-Bedienelement B14 befindet, der Nutzer U1 der Patientenlagerungsvorrichtung 10 und dem mobilen Steuerungsgerät 3 zugewandt ist und sich die Gantry 20 zur linken Hand des Nutzers U1 befindet.

Mit anderen Worten, kann die Zuordnung der horizontalen Richtungen für die Bewegung der Transferplatte 12 zu den Transfer-Bedienelementen B12 und B14 davon abhängen, auf welcher Seite in Bezug auf die Transferplatte 12 sich der Nutzer U1 befindet. Diese Zuordnung kann insbesondere dynamisch erfolgen, beispielsweise in dem die Pose des mobilen Steuerungsgeräts 3 regelmäßig in kurzen Zeitabständen ermittelt wird und die Zuordnung basierend auf der Pose angepasst wird. Die basierend auf der Pose des mobilen Steuerungsgeräts 3 ermittelte Zuordnung kann insbesondere mittels der Anzeigeeinheit 3Y angezeigt werden. Auf diese Weise kann der Nutzer U1 erkennen, welches der Transfer-Bedienelemente B12 und B14 zu betätigen ist, um die Transferplatte 12 in eine vorgegebene Richtung zu bewegen.

Alternativ oder zusätzlich kann jedes der Bedienelemente B10, B11, B12, B13, B14 jeweils berührungsempfindlich sein und/oder eine berührungsempfindliche Oberfläche aufweisen. Damit können die Bedienelemente B10, B11, B12, B13, B14 zusammen eine berührungsempfindliche Fläche bilden, welche zum Anzeigen zumindest eines Software-Bedienelements und/oder zum Erfassen zumindest einer Berührungsgeste zum Betätigen zumindest eines Software-Bedienelements ausgebildet ist. Das zumindest eine Software-Bedienelement kann insbesondere mittels der Anzeigeeinheit 3Y und/oder mittels des berührungsempfindlichen Bildschirms 5Y angezeigt werden.

Die berührungsempfindliche Fläche kann insbesondere aus den berührungsempfindlichen Oberflächen der Bedienelemente zusammengesetzt sein. Die berührungsempfindliche Fläche kann zusammenhängend sein oder aus mehreren voneinander separierten Teilflächen zusammengesetzt sein.

Mittels der berührungsempfindlichen Fläche kann insbesondere über mehrere Software-Elemente navigiert werden, beispielsweise um die Komponente der medizinischen Bildgebungsvorrichtung 2 zu steuern und/oder Daten, die dem Nutzer U1 angezeigt werden sollen, auszuwählen.

Mit Hilfe der zumindest einen Berührungsgeste, welche mittels der berührungsempfindlichen Fläche erfasst wird, kann insbesondere durch eine Liste mit Daten, welche beispielsweise den Patienten 13 und/oder ein Bildgebungsprotokoll und/oder ein Injektionsprotokoll betreffen, gescrollt werden. Das kann insbesondere durch ein Wischen mit den Fingern über die berührungsempfindliche Fläche, beispielsweise nach oben und/oder nach unten, erfolgen.

Mit Hilfe der zumindest einen Berührungsgeste, welche mittels der berührungsempfindlichen Fläche erfasst wird, kann insbesondere der jeweils nächste Schritt eines vorgegebenen Workflows, der beispielsweise die medizinische Bildgebungs-Untersuchung und/oder die Steuerung der medizinischen Bildgebungsvorrichtung 2 betrifft und/oder basierend auf dem Kontext von einer Steuerungssoftware automatisch ausgewählt wird, bestätigt oder storniert werden. Das kann insbesondere durch ein Wischen mit den Fingern über die berührungsempfindliche Fläche, beispielsweise nach rechts und/oder nach links, erfolgen. Auf diese Weise ist mit Hilfe des mobilen Steuerungsgeräts 3 eine Software-Navigation vorwärts und/oder rückwärts durch den Workflow realisierbar.

Unter einer medizinischen Bildgebungs-Untersuchung kann insbesondere eine medizinische Untersuchung verstanden werden, welche mittels der medizinischen Bildgebungsvorrichtung durchführbar und/oder zumindest teilweise durchgeführt ist.

Das mobile Steuerungsgerät 3 weist ferner die folgenden Komponenten auf:
- einen Anschluss C1 zur kabelgebundenen Stromversorgung,
- einen Anschluss C2 zur kabelgebundenen Datenübertragung,
- ein Datenübertragungsmodul 3T,
- ein Energieversorgungsmodul 3E,
- ein Datenverarbeitungsmodul 3P, welches ein Speichermodul und ein Prozessormodul aufweist, und
- eine Alarmsignal-Ausgabeeinheit 3A.

Statt der separaten Anschlüsse C1 und C2 kann beispielsweise ein Anschluss vorgesehen sein, mit dem Stromversorgung und Datenübertragung über dasselbe Kabel erfolgen können.

Insbesondere kann sich das Datenübertragungsmodul 3T ganz oder teilweise in dem Innenraum des mobilen Steuerungsgeräts 3 befinden. Insbesondere kann sich das Energieversorgungsmodul 3E ganz oder teilweise in dem Innenraum des mobilen Steuerungsgeräts 3 befinden. Insbesondere kann sich das Datenverarbeitungsmodul 3P ganz oder teilweise in dem Innenraum des mobilen Steuerungsgeräts 3 befinden. Insbesondere können sich eine oder mehrere weitere Komponenten ganz oder teilweise in dem Innenraum des mobilen Steuerungsgeräts 3 befinden.

Das mobile Steuerungsgerät 3 weist eine Totmanneinrichtung 3S auf, welche dazu ausgebildet ist, ein unbeabsichtigtes Betätigen des Bedienelements zu erkennen und/oder zu verhindern. Ein unbeabsichtigtes Betätigen des Bedienelements kann sich beispielsweise daraus ergeben, dass sich der Nutzer U1 versehentlich auf das mobile Steuerungsgerät 3 setzt.

Das mobile Steuerungsgerät 3 weist ein Andockmodul 3D auf. Das Andockmodul 3D kann insbesondere derart universell ausgebildet sein, dass das mobile Steuerungsgerät 3 mittels des Andockmoduls 3D sowohl lösbar an eine Andockeinheit der medizinischen Bildgebungsvorrichtung 2 andocken kann als auch mit dem Tablet und/oder mit der Trageeinheit verbunden werden kann.

Das mobile Steuerungsgerät 3 weist eine erste Audio-Schnittstelle mit einem Mikrophon A1 und einem Lautsprechen A2 auf, welche dazu ausgebildet ist, akustische Signale von dem Nutzer U1 zu empfangen und/oder an den Nutzer U1 auszugeben.

Basierend auf den akustischen Signalen, die mittels der ersten Audio-Schnittstelle von dem Nutzer U1 empfangen werden, können beispielsweise Sprachbefehle zur Steuerung der medizinischen Bildgebungsvorrichtung 2 ermittelt werden. Die Sprachbefehle können beispielsweise mittels einer Software zur Erkennung und Verarbeitung von Sprache ermittelt werden. Diese Software kann beispielsweise auf dem mobilen Steuerungsgerät 3 und/oder auf dem Tabletcomputer 5 und/oder auf der Steuerungsvorrichtung 30 installiert sein.

Mit Hilfe der ersten Audio-Schnittstelle kann der Nutzer U1 auch aus größerer Entfernung und/oder aus einem anderen Raum mit dem Patienten 13 und/oder mit anderen Personen in dem Untersuchungsraum sprechen, beispielsweise unter Verwendung einer zweiten Audio-Schnittstelle 40, welche an der Gantry 20 angeordnet sein kann und/oder welche einen Lautsprecher und/oder ein Mikrophon aufweisen kann.
Das mobile Steuerungsgerät 3 weist eine Anzeigeeinheit 3Y auf, welche dazu ausgebildet ist, dem Nutzer U1 Daten, welche die medizinische Bildgebungsvorrichtung 2 und/oder die Komponente der medizinischen Bildgebungsvorrichtung 2 und/oder die Untersuchung und/oder den Patienten 13 betreffen, anzuzeigen. Die Anzeigeeinheit 3Y kann beispielsweise als 7-Segmentanzeige und/oder als Bildschirm ausgebildet sein. Das mobile Steuerungsgerät 3 kann beispielsweise einen berührungsempfindlichen Bildschirm aufweisen, welcher die Anzeigeeinheit 3Y und eine Eingabeeinheit bildet. Insbesondere kann die Anzeigeeinheit 3Y in das Gehäuse 3C integriert sein. Mittels des berührungsempfindlichen Bildschirms kann beispielsweise eine Software zur Steuerung der medizinischen Bildgebungsvorrichtung 2 und/oder der Komponente der medizinischen Bildgebungsvorrichtung 2 bedient werden.

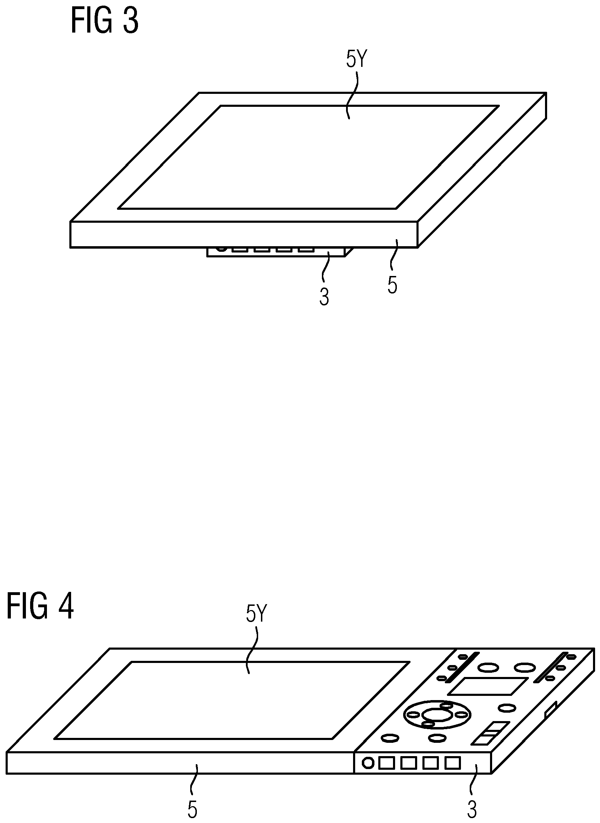

Fig. 2 zeigt ein mobiles Steuerungsgerät 3 eines Systems 1 gemäß einer zweiten Ausführungsform der Erfindung in einer ersten Ansicht. Fig. 3 zeigt ein mobiles Steuerungsgerät 3 eines Systems 1 gemäß der zweiten Ausführungsform der Erfindung in einer zweiten Ansicht.

Das mobile Steuerungsgerät 3 kann zusammen mit einem Tabletcomputer 5 und/oder einem Smartphone verwendet werden, auf dem eine Software-Applikation installiert ist, welche zum Steuern der medizinischen Bildgebungsvorrichtung 2 und/oder der Komponente der medizinischen Bildgebungsvorrichtung 2 ausgebildet ist.

Der Tabletcomputer 5 weist den berührungsempfindlichen Bildschirm 5Y auf. Mit Hilfe des Tabletcomputers können insbesondere eine oder mehrere der folgenden Schritte des Workflows einer medizinischen Bildgebungs-Untersuchung ausgeführt werden:
- Registrierung des Patienten 13,
- Anpassen von Untersuchungsparametern, beispielsweise des Bildgebungsprotokolls und/oder des Injektionsprotokolls,
- Bildverarbeitung und/oder Finalisierung von medizinischen Bilddatensätzen,
- Nutzung weiterer Radiologie-Software, insbesondere im Zusammenhang mit PACS, RIS, HIS,
- Kommunikation mit anderen Nutzern, insbesondere mit Mitarbeitern anderer Klinikabteilungen, beispielsweise Radiologe, Empfang, Notaufnahme oder ähnliches.

Das mobile Steuerungsgerät 3 kann beispielsweise magnetisch an den Tabletcomputer 5 und/oder an ein Smartphone angedockt werden. Der Tabletcomputer 5 und das mobile Steuerungsgerät 3 können auf diese Weise bequem mit nur einer Hand getragen werden. Das Smartphone und das mobile Steuerungsgerät 3 können auf diese Weise bequem mit nur einer Hand getragen werden.

Fig. 4 zeigt ein mobiles Steuerungsgerät 3 eines Systems 1 gemäß einer dritten Ausführungsform der Erfindung.

Das mobile Steuerungsgerät 3 kann beispielsweise den Tabletcomputer 5 aufweisen oder fest mit einem Tabletcomputer 5 zu einer zusammenhängenden Steuerungsbaugruppe verbunden werden. Die Steuerungsbaugruppe ermöglicht Interaktionen des Nutzers U1 mit der medizinischen Bildgebungsvorrichtung 2 sowohl mittels elektromechanischer Schaltelemente, welche insbesondere für sicherheitsrelevante Steuerungsbefehle vorteilhaft sein können, als auch mittels der auf dem Tabletcomputer 5 installierten Software-Applikation, welche insbesondere bei komplexeren Anwendungen, die nicht sicherheitskritisch sind, vorteilhaft sein kann.

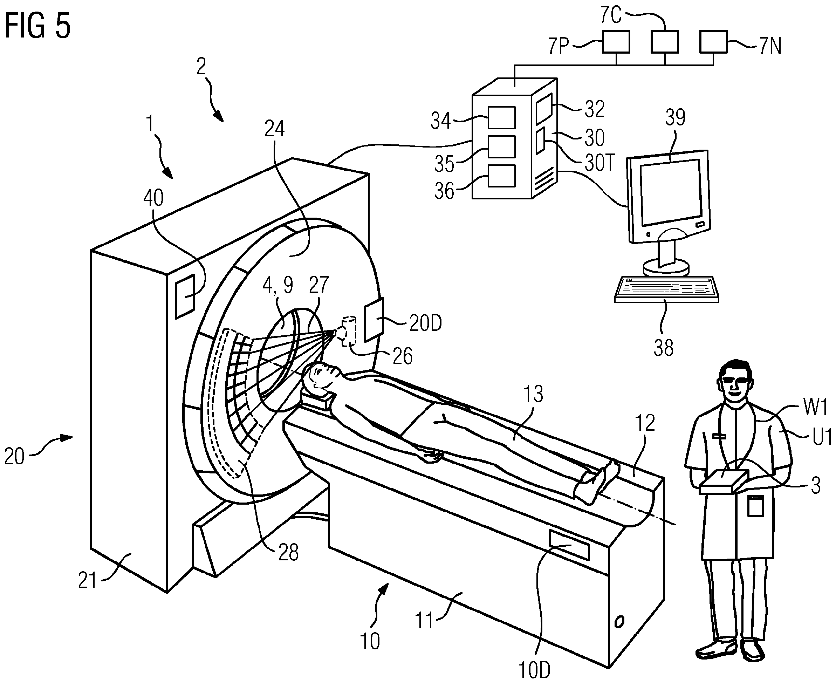

Fig. 5 zeigt ein System 1 gemäß einer vierten Ausführungsform der Erfindung, aufweisend die medizinische Bildgebungsvorrichtung 2.

Ohne Beschränkung des allgemeinen Erfindungsgedankens ist für die medizinische Bildgebungsvorrichtung 2 beispielhaft ein Computertomographiegerät gezeigt. Die medizinische Bildgebungsvorrichtung 2 weist die Gantry 20, die tunnelförmige Öffnung 9, die Patientenlagerungsvorrichtung 10 und die Steuerungsvorrichtung 30 auf.

Die Gantry 20 weist den stationären Tragrahmen 21 und den Rotor 24 auf. Der Rotor 24 ist mittels einer Drehlagerungsvorrichtung an einem Kipprahmen um eine Rotationsachse relativ zu dem stationären Tragrahmen 21 drehbar angeordnet.

In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungstisch 11 und die Transferplatte 12 zur Lagerung des Patienten 13 auf. Die Transferplatte 12 ist derart relativ zu dem Lagerungstisch 11 bewegbar an dem Lagerungstisch 11 angeordnet, dass die Transferplatte 12 in einer Längsrichtung der Transferplatte 12 in den Akquisitionsbereich 4 einführbar ist.

Die medizinische Bildgebungsvorrichtung 2 ist zur Akquisition von Akquisitionsdaten basierend auf einer elektromagnetischen Strahlung 27 ausgebildet. Die medizinische Bildgebungsvorrichtung 2 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor. Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 2 ausgebildet. Gemäß der vierten Ausführungsform der Erfindung ist die Steuerungseinheit 35 in die Steuerungsvorrichtung 30 integriert.

Die Steuerungsvorrichtung 30 wird von einem Datenverarbeitungssystem, welches einen Computer aufweist, gebildet und weist das computerlesbare Medium 32 und das Prozessorsystem 36 auf.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden.

Die medizinische Bildgebungsvorrichtung 2 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet.

Das System 1 weist eine Datenübertragungseinheit auf, welche zum Übertragen von Daten zwischen der Steuerungseinheit 35 und dem mobilen Steuerungsgerät 3 ausgebildet ist. Die Daten können beispielsweise Akquisitionsparameter, insbesondere Bildgebungsprotokoll-Parameter, Injektionsprotokollparameter, Patientenparameter, insbesondere aus der elektronischen Gesundheitsakte des Patienten 13, und/oder Messdaten aus Messungen zur Unterstützung der medizinischen Bildgebungs-Untersuchung, insbesondere aus einer EKG-Messung, umfassen. Die Datenübertragungseinheit weist das Datenübertragungsmodul 3T des mobilen Steuerungsgeräts 3 und das Datenübertragungsmodul 30T der Steuerungsvorrichtung 30 auf, wobei jedes der Datenübertragungsmodule 3T und 30T jeweils zum Senden und/oder Empfangen der Daten ausgebildet ist. Die Daten können beispielsweise kabellos und/oder kabelgebunden übertragen werden. Die Daten können insbesondere basierend auf einem Kommunikationsprotokoll übertragen werden, welches bidirektional und/oder erstfehlersicher ist.

Auf diese Weise ermöglicht die Erfindung eine kabellose sichere Fernsteuerung der medizinischen Bildgebungsvorrichtung 2 und/oder einer Komponente der medizinischen Bildgebungsvorrichtung 2. Durch das bidirektionale Kommunikationsprotokoll wird beispielsweise ermöglicht, dass zusätzlich zu den Daten, welche die Steuerung der Komponente betreffen und welche von dem mobilen Steuerungsgerät 3 an die Steuerungseinheit 35 übertragen werden, weitere Daten, welche beispielsweise den Zustand einer oder mehrerer Komponenten der medizinischen Bildgebungsvorrichtung 2 betreffen, an das mobile Steuerungsgerät 3 übertragen werden können. Damit können beispielsweise freigegebene Bewegungen der Transferplatte und/oder ein Zustand eines Laservisiers für die Patientenpositionierung mittels der Anzeigeeinheit des mobilen Steuerungsgeräts 3 dem Nutzer U1 angezeigt werden.

Das System 1 weist ferner die Kontext-Ermittlungseinheit 7C, die Posen-Ermittlungseinheit 7P und die Konfigurationseinheit 7N auf. Gemäß der vierten Ausführungsform der Erfindung weist das System 1 eine Trageinheit W1 mit einem Lanyard auf. Mit Hilfe des Lanyards trägt der Nutzer U1 das mobile Steuerungsgerät 3 am Körper. Die medizinische Bildgebungsvorrichtung 2 weist eine Andockeinheit 20D an der Gantry 20 und eine Andockeinheit 10D an der Patientenlagerungsvorrichtung 10 auf. Jede der Andockeinheiten 20D und 10D ist zum lösbaren Andocken des mobilen Steuerungsgeräts 3 ausgebildet.

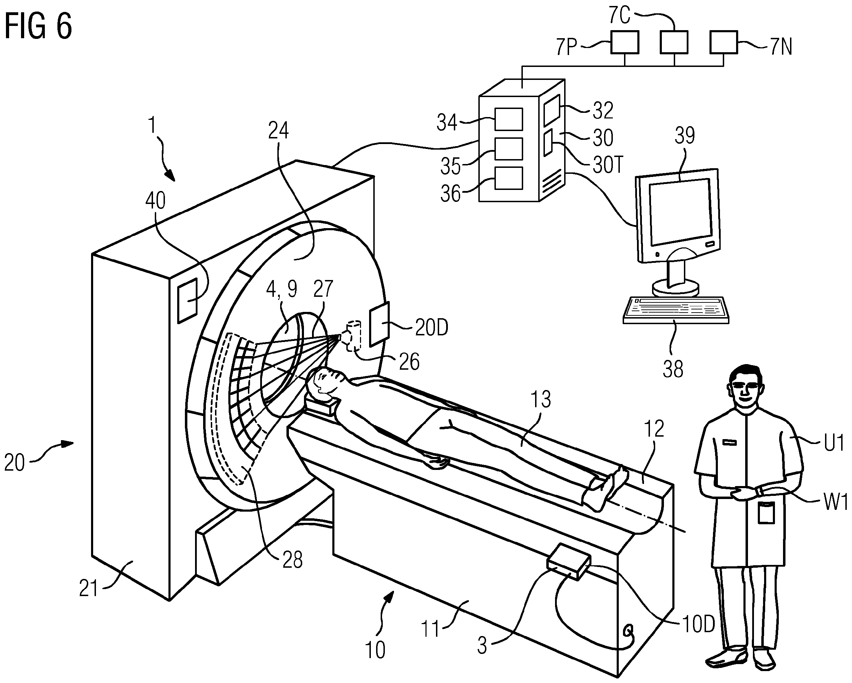

Fig. 6 zeigt ein System 1 gemäß einer fünften Ausführungsform der Erfindung, wobei das mobile Steuerungsgerät 3 an der Andockeinheit 10D angeordnet ist. Gemäß der fünften Ausführungsform der Erfindung ist das mobile Steuerungsgerät 3 mittels eines Kabels mit der medizinischen Bildgebungsvorrichtung 2 verbunden, wobei über das Kabel eine Stromversorgung und/oder eine Datenübertragung erfolgen kann.

Gemäß der fünften Ausführungsform der Erfindung weist das System 1 eine Trageinheit W1 mit einem Armband auf. Mit Hilfe des Armbands kann der Nutzer U1 das mobile Steuerungsgerät 3 am Körper, insbesondere am Handgelenk, tragen.

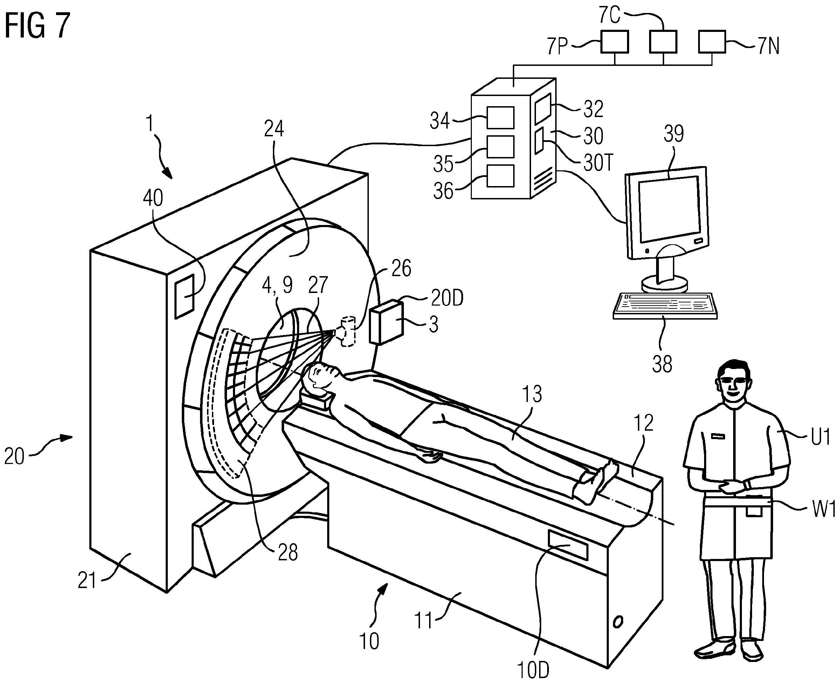

Fig. 7 zeigt ein System 1 gemäß einer sechsten Ausführungsform der Erfindung, wobei das mobile Steuerungsgerät 3 an der Andockeinheit 20D angeordnet ist. Gemäß der sechsten Ausführungsform der Erfindung weist das System 1 eine Trageinheit W1 mit einem Gürtel auf. Mit Hilfe des Gürtels kann der Nutzer U1 das mobile Steuerungsgerät 3 am Körper tragen.

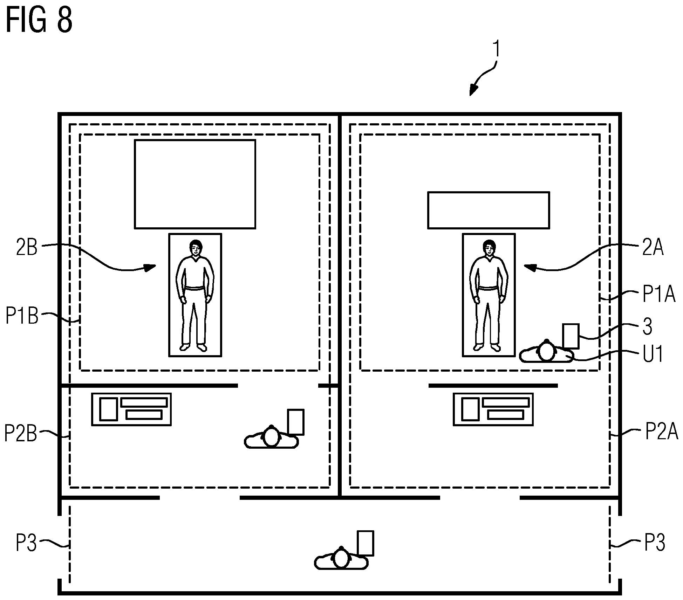

Fig. 8 ein System 1 gemäß einer siebenten Ausführungsform der Erfindung, wobei das System 1 zwei medizinische Bildgebungsvorrichtungen aufweist.

Das mobile Steuerungsgerät 3 kann beispielsweise in einem System 1 mit mehreren medizinischen Bildgebungsvorrichtungen verwendet werden. Die medizinischen Bildgebungsvorrichtungen können insbesondere unterschiedliche Bildgebungsmodalitäten, beispielsweise CT-Geräte und MR-Geräte, umfassen. Insbesondere kann das mobile Steuerungsgerät 3 dazu ausgebildet sein, zu erkennen, in welchem Raum und/oder in der Nähe welcher der mehreren medizinischen Bildgebungsvorrichtungen es sich befindet, und/oder sich mit der jeweiligen medizinischen Bildgebungsvorrichtung, insbesondere automatisch, beispielsweise gemäß "Plug & Play", zu verbinden.
Insbesondere kann mittels der Anzeigeeinheit 3Y des mobilen Steuerungsgeräts 3 angezeigt werden, mit welcher der mehreren medizinischen Bildgebungsvorrichtungen und/oder mit welcher der unterschiedlichen Bildgebungsmodalitäten das mobile Steuerungsgerät 3 verbunden ist.

Das System 1 kann insbesondere eine Mehrzahl von medizinischen Bildgebungsvorrichtungen und eine Auswahleinheit aufweisen, welche dazu ausgebildet ist, basierend auf dem Kontext und/oder basierend auf der Pose der mobilen Steuerungsbaugruppe eine medizinische Bildgebungsvorrichtung 2 aus der Mehrzahl von medizinischen Bildgebungsvorrichtungen auszuwählen. Die Auswahleinheit kann beispielsweise in das mobile Steuerungsgerät 3 und/oder in ein Datenverarbeitungssystem, welches insbesondere mit dem mobilen Steuerungsgerät 3 und/oder mit den Steuerungseinheiten der medizinischen Bildgebungsvorrichtungen mittels einer Datenübertragungsverbindung verbunden sein kann, integriert sein.

Insbesondere kann jede medizinische Bildgebungsvorrichtung der Mehrzahl von medizinischen Bildgebungsvorrichtungen jeweils eine Steuerungseinheit aufweisen, welche zum Ausgeben eines Steuersignals an eine Komponente der jeweiligen medizinischen Bildgebungsvorrichtung ausgebildet ist. Das System 1 kann insbesondere dazu ausgebildet sein, das mobile Steuerungsgerät 3 und die Steuerungseinheit der ausgewählten medizinischen Bildgebungsvorrichtung derart zu koppeln, dass ein Betätigen des Bedienelements des mobilen Steuerungsgeräts 3 das Ausgeben des Steuersignals an die Komponente mittels der Steuerungseinheit bewirkt.

Die Posen-Ermittlungseinheit 7P ist dazu ausgebildet, zu ermitteln, ob sich die Pose des mobilen Steuerungsgeräts 3 in einem ersten Posenbereich und/oder in einem zweiten Posenbereich befindet und/oder ob die Pose des mobilen Steuerungsgeräts 3 die Posenbereichsgrenze P3 überschreitet. Für die medizinische Bildgebungsvorrichtung 2A sind der erste Posenbereich P1A und der zweite Posenbereich P2A jeweils gestrichelt dargestellt. Für die medizinische Bildgebungsvorrichtung 2B sind der erste Posenbereich P1B und der zweite Posenbereich P2B jeweils gestrichelt dargestellt.

Gemäß der siebenten Ausführungsform der Erfindung ist vorgesehen, dass der erste Posenbereich den Untersuchungsraum, in dem die medizinische Bildgebungsvorrichtung installiert ist, umfasst und dass der zweite Posenbereich den Untersuchungsraum und den Kontrollraum, der von dem Untersuchungsraum mittels einer Trennwand zumindest teilweise abgetrennt ist, umfasst.

Gemäß der siebenten Ausführungsform der Erfindung wird durch ein Betätigen des entsprechenden Bedienelements, beispielsweise des ersten Transfer-Bedienelements oder des zweiten Transfer-Bedienelements, nur dann ein Ausgeben eines Steuersignals zum Bewegen der Transferplatte an die Patientenlagerungsvorrichtung bewirkt, wenn sich die Pose des mobilen Steuerungsgeräts 3 in dem entsprechenden ersten Posenbereich befindet.

Es können mehrere erste Posenbereiche, die jeweils einer Komponente und/oder einem Bedienelement zugeordnet sind, gewählt werden. Beispielweise kann ein anderer erster Posenbereich gewählt werden, der lediglich den Kontrollraum umfasst, wobei durch ein Betätigen des entsprechenden Bedienelements nur dann ein Ausgeben eines Steuersignals zum Auslösen der Strahlung an die Strahlungsquelle bewirkt wird, wenn sich die Pose des mobilen Steuerungsgeräts 3 in dem Kontrollraum befindet.

Gemäß der siebenten Ausführungsform der Erfindung sind das mobile Steuerungsgerät 3 und die Steuerungseinheit nur dann gekoppelt, wenn sich die Pose des mobilen Steuerungsgeräts 3 in dem entsprechenden zweiten Posenbereich befindet.

Gemäß der siebenten Ausführungsform der Erfindung wird, wenn die Pose des mobilen Steuerungsgeräts 3 die Posenbereichsgrenze P3 überschreitet, ein Alarmsignal mittels der Alarmsignal-Ausgabeeinheit 3A, beispielsweise in Form eines Alarmtons und/oder eines Vibrationsalarms, ausgegeben. Zum Ausschalten des Alarmsignals können beispielsweise ein erneutes Überschreiten der Posenbereichsgrenze P3 und/oder weitere Maßnahmen erforderlich sein. Auf diese Weise kann vermieden werden, dass das mobile Steuerungsgerät 3 versehentlich von einem Nutzer U1 mitgenommen wird.

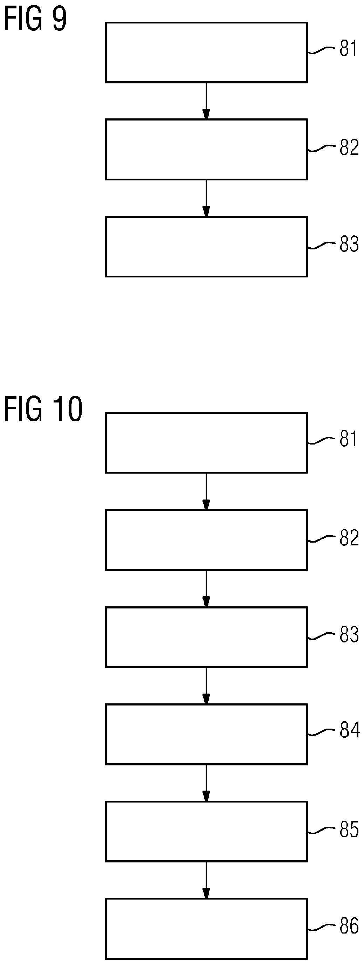

Fig. 9 zeigt ein Ablaufdiagramm für ein Verfahren zum Ausgeben eines Steuersignals an eine Komponente einer medizinischen Bildgebungsvorrichtung 2 gemäß einer achten Ausführungsform der Erfindung, wobei das Verfahren die folgenden Schritte umfasst:
- Koppeln 81 eines mobilen Steuerungsgeräts 3, welches ein Bedienelement aufweist, welches als ein elektromechanisches Schaltelement ausgebildet ist, und einer Steuerungseinheit 35, welche zum Ausgeben eines Steuersignals an die Komponente ausgebildet ist, derart, dass ein Betätigen des Bedienelements das Ausgeben des Steuersignals an die Komponente mittels der Steuerungseinheit 35 bewirkt,
- Betätigen 82 des Bedienelements,
- Ausgeben 83 des Steuersignals.

Fig. 10 zeigt ein Ablaufdiagramm für ein Verfahren zum Ausgeben eines Steuersignals an eine Komponente einer medizinischen Bildgebungsvorrichtung 2 gemäß einer neunten Ausführungsform der Erfindung, wobei das Verfahren ferner die folgenden Schritte umfasst:
- Ermitteln 84 eines Kontexts, welcher die medizinische Bildgebungsvorrichtung 2 und/oder eine medizinische Untersuchung, welche mittels der medizinischen Bildgebungsvorrichtung 2 durchführbar ist, betrifft, und/oder Ermitteln 85 einer Pose des mobilen Steuerungsgeräts 3,
- Konfigurieren 86 des mobilen Steuerungsgeräts 3 und/oder der Steuerungseinheit 35 basierend auf dem Kontext und/oder basierend auf der Pose des mobilen Steuerungsgeräts 3.

## Patentansprüche

1. System (1), aufweisend
- eine Komponente einer medizinischen Bildgebungsvorrichtung (2),
- ein mobiles Steuerungsgerät (3) mit einem Bedienelement, welches als ein elektromechanisches Schaltelement ausgebildet ist,
- eine Steuerungseinheit (35), welche zum Ausgeben eines Steuersignals an die Komponente ausgebildet ist,
- eine Posen-Ermittlungseinheit (7P), welche zum Ermitteln einer Pose des mobilen Steuerungsgeräts (3) ausgebildet ist,
- eine Konfigurationseinheit (7N), welche zum Konfigurieren des mobilen Steuerungsgeräts (3) und/oder der Steuerungseinheit (35) basierend auf der Pose des mobilen Steuerungsgeräts (3) ausgebildet ist,
- wobei in einem Betriebszustand des Systems (1) das mobile Steuerungsgerät (3) und die Steuerungseinheit (35) derart gekoppelt sind, dass ein Betätigen des Bedienelements das Ausgeben des Steuersignals an die Komponente mittels der Steuerungseinheit (35) bewirkt,
- wobei die Komponente der medizinischen Bildgebungsvorrichtung (2) einen Lagerungstisch (11) und eine Transferplatte (12) aufweist, welche relativ zu dem Lagerungstisch (11) bewegbar an dem Lagerungstisch (11) angeordnet ist,
- wobei das mobile Steuerungsgerät (3) ein erstes Transfer-Bedienelement aufweist,
- wobei das mobile Steuerungsgerät (3) und/oder die Steuerungseinheit (35) mittels der Konfigurationseinheit (7N) derart basierend auf der Pose des mobilen Steuerungsgeräts (3) konfigurierbar ist, dass für zumindest eine erste Pose des mobilen Steuerungsgeräts (3) ein Betätigen des ersten Transfer-Bedienelements ein Bewegen der Transferplatte (12) in eine erste horizontale Richtung bewirkt und dass für zumindest eine zweite Pose des mobilen Steuerungsgeräts (3) ein Betätigen des ersten Transfer-Bedienelements ein Bewegen der Transferplatte (12) in eine zweite horizontale Richtung bewirkt.

2. System (1) nach Anspruch 1,
- wobei das mobile Steuerungsgerät (3) derart ausgebildet ist, dass das mobile Steuerungsgerät (3) von einem Nutzer (U1) in einer Hand und/oder am Körper getragen werden kann und/oder dass das mobile Steuerungsgerät (3) von einem Nutzer (U1) bedient werden kann, während das mobile Steuerungsgerät (3) von dem Nutzer (U1) in einer Hand und/oder am Körper getragen wird.

3. System (1) nach einem der Ansprüche 1 bis 2,
- wobei das mobile Steuerungsgerät (3) eine erste Audio-Schnittstelle aufweist, welche dazu ausgebildet ist, akustische Signale von dem Nutzer (U1) zu empfangen und/oder an den Nutzer (U1) auszugeben.

4. System (1) nach einem der Ansprüche 1 bis 3,
- wobei das mobile Steuerungsgerät (3) mehrere berührungsempfindliche Bedienelemente aufweist, welche zusammen eine berührungsempfindliche Fläche bilden, welche zum Anzeigen zumindest eines Software-Bedienelements und/oder zum Erfassen zumindest einer Berührungsgeste zum Betätigen zumindest eines Software-Bedienelements ausgebildet ist.

5. System (1) nach einem der Ansprüche 1 bis 4,
- wobei das mobile Steuerungsgerät (3) eine Anzeigeeinheit (3Y) aufweist, welche dazu ausgebildet ist, dem Nutzer (U1) Daten, welche die medizinische Bildgebungsvorrichtung (2) und/oder die Komponente der medizinischen Bildgebungsvorrichtung (2) betreffen, anzuzeigen.

6. System (1) nach einem der Ansprüche 1 bis 5, ferner aufweisend
- zumindest eine Kontext-Ermittlungseinheit (7C), welche zum Ermitteln eines Kontexts ausgebildet ist, welcher die medizinische Bildgebungsvorrichtung (2) und/oder eine medizinische Untersuchung, welche mittels der medizinischen Bildgebungsvorrichtung (2) durchführbar ist, betrifft.

7. System (1) nach Anspruch 6,
- wobei die Posen-Ermittlungseinheit (7P) dazu ausgebildet ist, zu ermitteln, ob die Pose des mobilen Steuerungsgeräts (3) eine Posenbereichsgrenze überschreitet,
- wobei das System (1) eine Alarmsignal-Ausgabeeinheit aufweist, welche zum Ausgeben eines Alarmsignals derart ausgebildet ist, dass das Alarmsignal ausgegeben wird, wenn die Pose des mobilen Steuerungsgeräts (3) eine Posenbereichsgrenze überschreitet.

8. System (1) nach einem der Ansprüche 6 bis 7,
- wobei die Konfigurationseinheit (7N) zum Konfigurieren des mobilen Steuerungsgeräts (3) und/oder der Steuerungseinheit (35) basierend auf dem Kontext ausgebildet ist.

9. System (1) nach Anspruch 8,
- wobei die Posen-Ermittlungseinheit (7P) dazu ausgebildet ist, zu ermitteln, ob sich die Pose des mobilen Steuerungsgeräts (3) in einem ersten Posenbereich befindet,
- wobei das mobile Steuerungsgerät (3) und/oder die Steuerungseinheit (35) mittels der Konfigurationseinheit (7N) derart basierend auf der Pose des mobilen Steuerungsgeräts (3) konfigurierbar ist, dass ein Betätigen des Bedienelements das Ausgeben des Steuersignals an die Komponente mittels der Steuerungseinheit (35) in Abhängigkeit davon bewirkt, ob sich die Pose in dem ersten Posenbereich befindet.

10. System (1) nach einem der Ansprüche 8 bis 9,
- wobei die Posen-Ermittlungseinheit (7P) dazu ausgebildet ist, zu ermitteln, ob sich die Pose des mobilen Steuerungsgeräts (3) in einem zweiten Posenbereich befindet,
- wobei das mobile Steuerungsgerät (3) und/oder die Steuerungseinheit (35) mittels der Konfigurationseinheit (7N) derart basierend auf der Pose des mobilen Steuerungsgeräts (3) konfigurierbar ist, dass das mobile Steuerungsgerät (3) und die Steuerungseinheit (35) in Abhängigkeit davon gekoppelt sind, ob sich die Pose in dem zweiten Posenbereich befindet.

11. System (1) nach einem der Ansprüche 1 bis 10, ferner aufweisend
- einen Tabletcomputer (5) und/oder ein Smartphone mit einer Software-Applikation, welche zum Steuern der medizinischen Bildgebungsvorrichtung (2) und/oder der Komponente der medizinischen Bildgebungsvorrichtung (2) ausgebildet ist,
- wobei das mobile Steuerungsgerät (3) den Tabletcomputer (5) und/oder das Smartphone aufweist und/oder wobei das mobile Steuerungsgerät (3) mit dem Tabletcomputer (5) und/oder mit dem Smartphone mittels einer mechanischen Verbindung und/oder mittels einer Datenübertragungsverbindung verbindbar ist.

12. System (1) nach einem der Ansprüche 1 bis 11, ferner aufweisend
- die medizinische Bildgebungsvorrichtung (2),
- wobei die medizinische Bildgebungsvorrichtung (2) eine Andockeinheit aufweist, welche zum lösbaren Andocken des mobilen Steuerungsgeräts (3) ausgebildet ist.

13. System (1) nach einem der Ansprüche 11 bis 12,
- wobei die mechanische Verbindung und/oder die Andockeinheit eine magnetische Halterung aufweist.

14. Verfahren zum Ausgeben eines Steuersignals an eine Komponente einer medizinischen Bildgebungsvorrichtung (2), wobei das Verfahren die folgenden Schritte umfasst:
- Koppeln (81) eines mobilen Steuerungsgeräts (3), welches ein Bedienelement aufweist, welches als ein elektromechanisches Schaltelement ausgebildet ist, und einer Steuerungseinheit (35), welche zum Ausgeben eines Steuersignals an die Komponente ausgebildet ist, derart, dass ein Betätigen des Bedienelements das Ausgeben des Steuersignals an die Komponente mittels der Steuerungseinheit (35) bewirkt,
- Betätigen (82) des Bedienelements,
- Ausgeben (83) des Steuersignals,
- Ermitteln (85) einer Pose des mobilen Steuerungsgeräts (3),
- Konfigurieren (86) des mobilen Steuerungsgeräts (3) und/oder der Steuerungseinheit (35) basierend auf der Pose des mobilen Steuerungsgeräts (3),
- wobei die Komponente der medizinischen Bildgebungsvorrichtung (2) einen Lagerungstisch (11) und eine Transferplatte (12) aufweist, welche relativ zu dem Lagerungstisch (11) bewegbar an dem Lagerungstisch (11) angeordnet ist,
- wobei das mobile Steuerungsgerät (3) ein erstes Transfer-Bedienelement aufweist,
- wobei das mobile Steuerungsgerät (3) und/oder die Steuerungseinheit (35) mittels der Konfigurationseinheit (7N) derart basierend auf der Pose des mobilen Steuerungsgeräts (3) konfigurierbar ist, dass für zumindest eine erste Pose des mobilen Steuerungsgeräts (3) ein Betätigen des ersten Transfer-Bedienelements ein Bewegen der Transferplatte (12) in eine erste horizontale Richtung bewirkt und dass für zumindest eine zweite Pose des mobilen Steuerungsgeräts (3) ein Betätigen des ersten Transfer-Bedienelements ein Bewegen der Transferplatte (12) in eine zweite horizontale Richtung bewirkt.

15. Verfahren nach Anspruch 14, ferner umfassend die folgenden Schritte:
- Ermitteln (84) eines Kontexts, welcher die medizinische Bildgebungsvorrichtung (2) und/oder eine medizinische Untersuchung, welche mittels der medizinischen Bildgebungsvorrichtung (2) durchführbar ist, betrifft,
- Konfigurieren (86) des mobilen Steuerungsgeräts (3) und/oder der Steuerungseinheit (35) basierend auf dem Kontext.

16. Verwendung eines Systems (1) nach einem der Ansprüche 1 bis 13 zum Ausgeben eines Steuersignals an eine Komponente einer medizinischen Bildgebungsvorrichtung (2),
- wobei das Ausgeben des Steuersignals an die Komponente mittels der Steuerungseinheit (35) bewirkt wird, indem das Bedienelement betätigt wird.

## Claims

1. System (1), having
- a component of a medical imaging apparatus (2),
- a mobile control device (3) having an operating element which is designed as an electromechanical switching element,
- a control unit (35) which is designed in order to output a control signal to the component,
- a pose determination unit (7P) which is designed in order to determine a pose of the mobile control device (3),
- a configuration unit (7N) which is designed in order to configure the mobile control device (3) and/or the control unit (35) on the basis of the pose of the mobile control device (3),
- wherein, when the system is in an operating state (1), the mobile control device (3) and the control unit (35) are coupled such that an actuation of the operating element causes the control signal to be output to the component by means of the control unit (35),
- wherein the component of the medical imaging apparatus (2) has an examination table (11) and a transfer plate (12) which is arranged on the examination table (11) and can be moved relative to the examination table (11),
- wherein the mobile control device (3) has a first transfer operating element,
- wherein the mobile control device (3) and/or the control unit (35) can be configured by means of the configuration unit (7N) in such a manner on the basis of the pose of the mobile control device (3) that for at least a first pose of the mobile control device (3) an actuation of the first transfer operating element causes the transfer plate (12) to move in a first horizontal direction and that for at least a second pose of the mobile control device (3) an actuation of the first transfer operating element causes the transfer plate (12) to move in a second horizontal direction.

2. System (1) according to claim 1,
- wherein the mobile control device (3) is designed in such a manner that the mobile control device (3) can be carried in one hand or on the body by a user (U1) and/or that the mobile control device (3) can be operated by a user (U1) while the mobile control device (3) is being carried in one hand and/or on the body by the user (U1).

3. System (1) according to one of claims 1 to 2,
- wherein the mobile control device (3) has a first audio interface which is designed in order to receive acoustic signals from the user (U1) and/or to output acoustic signals to the user (U1).

4. System (1) according to one of claims 1 to 3,
- wherein the mobile control device (3) has a plurality of touch-sensitive operating elements which together form a touch-sensitive panel that is designed in order to display at least one software operating element and/or to capture at least one touch gesture for actuating at least one software operating element.

5. System (1) according to one of claims 1 to 4,
- wherein the mobile control device (3) has a display unit (3Y) which is designed in order to display to the user (U1) data relating to the medical imaging apparatus (2) and/or the component of the medical imaging apparatus (2).

6. System (1) according to one of claims 1 to 5, further having
- at least one context determination unit (7C) which is designed in order to determine a context that relates to the medical imaging apparatus (2) and/or a medical examination which can be carried out by means of the medical imaging apparatus (2).

7. System (1) according to claim 6,
- wherein the pose determination unit (7P) is designed in order to determine whether the pose of the mobile control device (3) crosses a pose region boundary,
- wherein the system (1) has an alarm signal output unit which is designed in order to output an alarm signal in such a manner that the alarm signal is output when the pose of the mobile control device (3) crosses a pose region boundary.

8. System (1) according to one of claims 6 to 7,
- wherein the configuration unit (7N) is designed in order to configure the mobile control device (3) and/or the control unit (35) on the basis of the context.

9. System (1) according to claim 8,
- wherein the pose determination unit (7P) is designed in order to determine whether the pose of the mobile control device (3) is situated in a first pose region,
- wherein the mobile control device (3) and/or the control unit (35) can be configured by means of the configuration unit (7N) in such a manner on the basis of the pose of the mobile control device (3) that an actuation of the operating element causes the control signal to be output to the component by means of the control unit (35) depending on whether the pose is situated in the first pose region.

10. System (1) according to one of claims 8 to 9,
- wherein the pose determination unit (7P) is designed in order to determine whether the pose of the mobile control device (3) is situated in a second pose region,
- wherein the mobile control device (3) and/or the control unit (35) can be configured by means of the configuration unit (7N) in such a manner on the basis of the pose of the mobile control device (3) that the mobile control device (3) and the control unit (35) are coupled depending on whether the pose is situated in the second pose region.

11. System (1) according to one of claims 1 to 10, further having
- a tablet computer (5) and/or a smartphone having a software application which is designed in order to control the medical imaging apparatus (2) and/or the component of the medical imaging apparatus (2),
- wherein the mobile control device (3) has the tablet computer (5) and/or the smartphone and/or wherein the mobile control device (3) can be connected to the tablet computer (5) and/or to the smartphone by means of a mechanical connection and/or by means of a data transfer connection.

12. System (1) according to one of claims 1 to 11, further having
- the medical imaging apparatus (2),
- wherein the medical imaging apparatus (2) has a docking unit which is designed in order to dock the mobile control device (3) in releasable fashion.

13. System (1) according to one of claims 11 to 12,
- wherein the mechanical connection and/or the docking unit has a magnetic holder.

14. Method for outputting a control signal to a component of a medical imaging apparatus (2), wherein the method comprises the following steps:
- coupling (81) of a mobile control device (3), which has an operating element that is designed as an electromechanical switching element, and a control unit (35), which is designed in order to output a control signal to the component, in such a manner that an actuation of the operating element causes the control signal to be output to the component by means of the control unit (35),
- actuation (82) of the operating element,
- output (83) of the control signal,
- determination (85) of a pose of the mobile control device (3),
- configuration (86) of the mobile control device (3) and/or of the control unit (35) on the basis of the pose of the mobile control device (3),
- wherein the component of the medical imaging apparatus (2) has an examination table (11) and a transfer plate (12) which is arranged on the examination table (11) and can be moved relative to the examination table (11),
- wherein the mobile control device (3) has a first transfer operating element,
- wherein the mobile control device (3) and/or the control unit (25) can be configured by means of the configuration unit (7N) in such a manner on the basis of the pose of the mobile control device (3) that for at least a first pose of the mobile control device (3) an actuation of the first transfer operating element causes the transfer plate (12) to move in a first horizontal direction and that for at least a second pose of the mobile control device (3) an actuation of the first transfer operating element causes the transfer plate (12) to move in a second horizontal direction.

15. Method according to claim 14, further comprising the following steps:
- determination (84) of a context which relates to the medical imaging apparatus (2) and/or a medical examination that can be carried out by means of the medical imaging apparatus (2),
- configuration (86) of the mobile control device (3) and/or of the control unit (35) on the basis of the context.

16. Use of a system (1) according to one of claims 1 to 13 in order to output a control signal to a component of a medical imaging apparatus (2),
- wherein the output of the control signal to the component by means of the control unit (35) is effected by actuating the operating element.

## Revendications

1. Système (1), comportant
- un composant d'une installation (2) d'imagerie médicale,
- un appareil (3) mobile de commande, ayant un élément de manœuvre constitué sous la forme d'un élément électromécanique de commutation,
- une unité (35) de commande, constituée pour envoyer un signal de commande au composant,
- une unité (7P) de détermination de pose, constituée pour déterminer une pose de l'appareil (3) mobile de commande,
- une unité (7N) de configuration, constituée pour configurer l'appareil (3) mobile de commande et/ou l'unité (35) de commande sur la base de la pose de l'appareil (3) mobile de commande,
- dans lequel, dans un état de fonctionnement du système (1), l'appareil (3) mobile de commande et l'unité (35) de commande sont couplés de manière à ce qu'un actionnement de l'élément de manœuvre provoque l'envoi du signal de commande au composant au moyen de l'unité (35) de commande,
- dans lequel le composant de l'installation (2) d'imagerie médicale a une table (11) de mise en position et un plateau (12) de transfert monté sur la table (11) de mise en position, en étant mobile par rapport à la table (11) de mise en position,
- dans lequel l'appareil (3) mobile de commande a un premier élément de manœuvre de transfert,
- dans lequel l'appareil (3) mobile de commande et/ou l'unité (35) de commande peut, au moyen de l'unité (7N) de configuration, être configuré sur la base de la pose de l'appareil (3) mobile de commande, de manière à ce que, pour au moins une première pose de l'appareil (3) mobile de commande, un actionnement du premier élément de manœuvre de transfert provoque un déplacement du plateau (12) de transfert dans une première direction horizontale et de manière à ce que, pour au moins une deuxième pose de l'appareil (3) mobile de commande, un actionnement du premier élément de manœuvre de transfert provoque un déplacement du plateau (12) de transfert dans une deuxième direction horizontale.

2. Système (1) suivant la revendication 1,
- dans lequel l'appareil (3) mobile de commande est constitué de manière à ce que l'appareil (3) mobile de commande puisse être porté à la main et/ou au corps par un utilisateur (U1) et/ou de manière à ce que l'appareil (3) mobile de commande puisse être manœuvré par un utilisateur (U1) pendant que l'appareil (3) mobile de commande est porté à la main et/ou au corps par l'utilisateur (U1).

3. Système (1) suivant l'une des revendications 1 à 2,
- dans lequel l'appareil (3) mobile de commande a une première interface audio, constituée pour recevoir des signaux acoustiques de l'utilisateur (U1) et/ou pour en envoyer à l'utilisateur (U1).

4. Système (1) suivant l'une des revendications 1 à 3,
- dans lequel l'appareil (3) mobile de commande a plusieurs éléments de manœuvre sensibles au toucher, qui forment ensemble une surface sensible au toucher, constituée pour l'affichage d'au moins un élément de manœuvre de logiciel et/ou pour la détection d'au moins un geste de toucher pour l'actionnement d'au moins un élément de manœuvre de logiciel.

5. Système (1) suivant l'une des revendications 1 à 4,
- dans lequel l'appareil (3) mobile de commande a une unité (3Y) d'affichage, constituée pour afficher à l'utilisateur (U1) des données, qui concernent l'installation (2) d'imagerie médicale et/ou le composant de l'installation (2) d'imagerie médicale.

6. Système (1) suivant l'une des revendications 1 à 5, comportant, en outre
- au moins une unité (7C) de détermination de contexte, constituée pour déterminer un contexte, qui concerne l'installation (2) d'imagerie médicale et/ou un examen médical pouvant être effectué au moyen de l'installation (2) d'imagerie médicale.

7. Système (1) suivant la revendication 6,
- dans lequel l'unité (7P) de détermination de pose est constituée pour déterminer si la pose de l'appareil (3) mobile de commande dépasse une limite de plage de pose,
- dans lequel le système (1) a une unité d'émission d'un signal d'alerte, constitué pour émettre un signal d'alerte, de manière à émettre le signal d'alerte si la pose de l'appareil (3) mobile de commande dépasse une limite de plage de pose.

8. Système (1) suivant l'une des revendications 6 à 7,
- dans lequel l'unité (7N) de configuration est constituée pour configurer l'appareil (3) mobile de commande et/ou l'unité (35) de commande sur la base du contexte.

9. Système (1) suivant la revendication 8,
- dans lequel l'unité (7P) de détermination de pose est constituée pour déterminer si la pose de l'appareil (3) mobile de commande se trouve dans une première plage de pose ,
- dans lequel l'appareil (3) mobile de commande et/ou l'unité (35) de commande peut, au moyen de l'unité (7N) de configuration, être configuré sur la base de la pose de l'appareil (3) mobile de commande, de manière à ce qu'un actionnement de l'élément de manœuvre provoque l'envoi du signal de commande au composant, si la pose se trouve dans la première plage de pose.

10. Système (1) suivant l'une des revendications 8 à 9,
- dans lequel l'unité (7P) de détermination de pose est constituée pour déterminer si la pose de l'appareil (3) mobile de commande se trouve dans une deuxième plage de pose,
- dans lequel l'appareil (3) mobile de commande et/ou l'unité (35) de commande peut, au moyen de l'unité (7N) de configuration, être configuré sur la base de la pose de l'appareil (3) mobile de commande, de manière à ce que l'appareil (3) mobile de commande et l'unité (35) de commande soient couplés, si la pose se trouve dans la deuxième plage de pose.

11. Système (1) suivant l'une des revendications 1 à 10, comportant, en outre
- un ordinateur (5) à tablette et/ou un téléphone intelligent, ayant une application de logiciel constituée pour commander l'installation (2) d'imagerie médicale et/ou le composant de l'installation (2) d'imagerie médicale,
- dans lequel l'appareil (3) mobile de commande a l'ordinateur (5) à tablette et/ou le téléphone intelligent et/ou dans lequel l'appareil (3) mobile de commande peut être relié à l'ordinateur (5) à tablette et/ou au téléphone intelligent au moyen d'une liaison mécanique et/ou au moyen d'une liaison de transmission de données.

12. Système (1) suivant l'une des revendications 1 à 11, comportant, en outre
- l'installation (2) d'imagerie médicale,
- dans lequel l'installation (2) d'imagerie médicale a une unité d'approche constituée pour l'approche détachable de l'appareil (3) mobile de commande.

13. Système (1) suivant l'une des revendications 11 à 12,
- dans lequel la liaison mécanique et/ou l'unité d'approche a une fixation magnétique.

14. Procédé d'envoi d'un signal de commande à un composant d'une installation (2) d'imagerie médicale, le procédé comprenant les stades suivants :
- couplage (81) d'un appareil (3) mobile de commande, qui a un élément de manœuvre, constitué sous la forme d'un élément électromécanique de commutation, et d'une unité (35) de commande, constituée pour envoyer un signal de commande au composant, de manière à ce qu'un actionnement de l'élément de manœuvre provoque l'envoi du signal de commande au composant, au moyen de l'unité (35) de commande,
- actionnement (82) de l'élément de manœuvre,
- envoi (83) du signal de commande,
- détermination (85) d'une pose de l'appareil (3) mobile de commande,
- configuration (86) de l'appareil (3) mobile de commande et/ou de l'unité (35) de commande sur la base de la pose de l'appareil (3) mobile de commande,
- dans lequel le composant de l'installation (2) d'imagerie médicale a une table (11) de mise en position et un plateau (12) de transfert monté sur la table (11) de mise en position, en étant mobile par rapport à la table (11) de mise en position,
- dans lequel l'appareil (3) mobile de commande a un premier élément de manœuvre de transfert,
- dans lequel l'appareil (3) mobile de commande et/ou l'unité (35) de commande peut, au moyen de l'unité (7N) de configuration, être configuré sur la base de la pose de l'appareil (3) mobile de commande, de manière à ce que, pour au moins une première pose de l'appareil (3) mobile de commande, un actionnement du premier élément de manœuvre de transfert provoque un déplacement du plateau (12) de transfert dans une première direction horizontale et de manière à ce que, pour au moins une deuxième pose de l'appareil (3) mobile de commande, un actionnement du premier élément de manœuvre de transfert provoque un déplacement du plateau (12) de transfert dans une deuxième direction horizontale.

15. Procédé suivant la revendication 14, comprenant, en outre, les stades suivants :
- détermination (84) d'un contexte, qui concerne l'installation (2) d'imagerie médicale et/ou un examen médical pouvant être effectué au moyen de l'installation (2) d'imagerie médicale,
- configuration (86) de l'appareil (3) mobile de commande et/ou de l'unité (35) de commande sur la base du contexte.

16. Utilisation d'un système (1) suivant l'une des revendications 1 à 13, pour envoyer un signal de commande à un composant d'une installation (2) d'imagerie médicale,
- dans lequel l'envoi du signal de commande au composant est provoqué au moyen de l'unité (35) de commande en actionnant l'élément de manœuvre.
